# EUROPÄISCHE PATENTANMELDUNG

(11) **EP 1 719 502 A1**
(43) Veröffentlichungstag der Anmeldung: **08.11.2006**
(21) Anmeldenummer: 06007758.3
(22) Anmeldetag: 21.01.2003
(51) Int. Cl.: A61K 9/06, A61K 9/10, A61K 47/32, A61K 47/38

(54) **Zusammensetzung zur Erleichterung der Humangeburt**

(30) Priorität: 22.01.2002 CH 1212002
(62) Teilanmeldung aus: 03722326.0
(71) Anmelder: HCB Happy Child Birth Holding AG, 4052 Basel (CH)
(72) Erfinder: Schaub, Andreas.F., CH-6318 Walchwill (CH)
(74) Vertreter: Weiss, Wolfgang

(57) **Zusammenfassung**

Verwendung einer physiologisch unbedenklichen organischen Substanz zur Herstellung einer Zusammensetzung ohne Gehalt an Alkalimetallsalzen von Metaphosphaten zur Anwendung als Gleitmittel bei Vaginalgeburten von Frauen. Mit der Zusammensetzung kann der Geburtsvorgang erleichtert und beschleunigt werden. Mit beigemischten pharmazeutischen Wirkstoffen kann zusätzlich zum Beispiel Infektionen vorgebeugt und das Schmerzempfinden beeinflusst werden.

## Beschreibung

Die vorliegende Erfindung betrifft die Verwendung einer physiologisch unbedenklichen organischen Substanz zur Herstellung einer Zusammensetzung ohne Gehalt an Alkalimetallsalzen von Metaphosphaten zur Anwendung als Gleitmittel bei Vaginalgeburten von Frauen. Die Gleitwirkung wird hierbei mittels Einbringen der Zusammensetzung über die Vagina in den Geburtskanal beziehungsweise der Bildung einer Schicht auf im wesentlichen der gesamten Oberfläche des Geburtskanals oder Teilen der Oberfläche des Geburtskanals er zielt.

Die vaginale Geburt eines Kindes ist ein komplexer Vorgang und wird durch drei wesentliche Faktoren bestimmt: dem Geburtsobjekt (Fötus, Amnion, Plazenta), dem Geburtskanal (bestehend aus einem knöchernen Anteil und einem Weichteilschlauch) und den Geburtskräften. Aus der wissenschaftlichen Fachliteratur sind verschiedene Geburtskräfte bekannt, welche die vaginale Geburt eines menschlichen Föten fördern oder hemmen. Geburtsfördernde Kräfte sind hierbei die Wehen und die Schwerkraft, geburtshemmende Kräfte sind die Dehnkraft des Muttermunds und des Geburtskanals. Die Geburt eines menschlichen Kindes wird in 3 Phasen eingeteilt: die Eröffnungsperiode, die Austreibungsperiode und die Plazentaperiode. Die normale Geburtsdauer beträgt bei Erstgebärenden im Durchschnitt 12 Stunden, bei Mehrgebärenden im Durchschnitt 8 Stunden. Die kürzere durchschnittliche Geburtsdauer bei Mehrgebärenden im Vergleich zu Erstgebärenden wird mit der verminderten Dehnkraft des Geburtskanals begründet, da bei Mehrgebärenden der Weichteilschlauch [inneres Weichteilrohr (Uterinsegment - Zervixkanal - Weichteilansatzrohr (Scheide und Vulva)] durch die vorangegangenen Vaginalgeburten ausgewalzt ist. Herrschende Lehrmeinung in Bezug auf die Geburtsmechanik beim Menschen ist folglich, dass die Dehnkraft des Geburtskanals (die Kraft, welche notwendig ist, um den Geburtskanal zu öffnen, zu dehnen und auszuwalzen) als wesentliche, die Geburt hindernde Kraft anzusehen ist (Dudenhausen, Schneider, Frauenheilkunde und Geburtshilfe, Verlag De Gruyter (1994), Seiten 113 bis 121).

In der Veterinärmedizin ist die geburtsmechanische Bedeutung der Reibungskraft zwischen Geburtsobjekt und Geburtskanal seit Jahrzehnten bekannt. Die Schmierung des Geburtskanals zur Reduktion dieser Reibungskraft ist in der veterinärmedizinischen Geburtshilfe eine Standardmethode (Richter, Götze; Tiergeburtshilfe, 4. Auflage; Verlag Paul Parey; Rechtsfragen in der Tiergeburtshilfe, Seite 614), und Schmiermittel für diesen Zweck sind im Handel erhältlich. Bei der Tiergeburt können relativ hohe Volumina an Schmiermitteln eingesetzt werden. Hierdurch ergibt sich die Möglichkeit, flüssige, wässrige Formulierungen zu verwenden, die als Ersatz für das schmierfähige Fruchtwasser dienen. Derart hohe Volumina können mangels Praktikabilität bei der menschlichen Vaginalgeburt nicht angewendet werden.

Ein wesentlicher Unterschied zwischen der Geburt beim Tier und der Geburt beim Menschen ist, dass die Rolle des Fruchtwassers bei der menschlichen Geburt am Termin keine signifikante Relevanz in Bezug auf die Schmierung des Geburtskanals hat, sondern die resultierenden Reibungskräfte erhöhen kann. Fruchtwasser hat als wässrige Substanz beim Menschen per se keinen ausgeprägten Schmiereffekt. Heute werden beim Menschen zumeist nur noch Geburten aus Schädellage vaginal durchgeführt, bei welchen der Abgang von Fruchtwasser unter der Geburt aufgrund der Abdichtung durch den Kopf nur als unwesentlich zu bezeichnen ist. Die Vernix Caseosa, als einzige schmierende Substanz des Geburtsobjekt, ist zumeist um den Geburtstermin nicht mehr vorhanden und wird ohnehin am Kopf nur wenig wirksam. Die Verwendung von Fruchtwasser oder Fruchtwasserersatz zur Schmierung des Geburtskanals vor oder während einer Vaginalgeburt beim Menschen ist daher keine taugliche Massnahme zur Verminderung der Reibungskräfte und zur Erleichterung der Vaginalgeburt beim Menschen.

Auch beim Menschen wird teilweise die Bedeutung der Reibungskraft zwischen Fötus und Geburtskanal als geburtshindemde Kraft diskutiert. So hat beispielsweise Moolgaoker in einer Untersuchung bei der Zangengeburt festgestellt, dass der Erfolg der Zangengeburt auch wesentlich durch diese Reibungskraft beeinflusst ist [Moolgaoker et al., Obstet. Gynecol. 1979; 54(3), Seiten 299-309]. Auch Cherkassky hat teilweise die Bedeutung der Reibungskraft während eines vaginaloperativen Eingriffs erkannt und hat daher in einem Patent vorgeschlagen, die Reibungskraft des Geburtskanals während eines vaginaloperativen Eingriffs durch die Einlage von Plastikfolien in die Vagina zu reduzieren und dadurch die Zangengeburt zu erleichtern (siehe US-A-4,602,623).

In RU-C2-2177789 wird beschrieben, bei der Einleitung der Geburt den Gebärmuttermund mit einem Natrium-Hyaluronat enthaltenden Gel zu behandeln, um eine Erweichung und Öffnung (Reifung) des Gebärmuttermundes zu erzielen. Die Reibung im Geburtskanal wird nicht angesprochen.

In US-A-3,614,797 wird vorgeschlagen, eine Zusammensetzung aus Kaliummetaphosphat, Alginat, Carboxymethylcellulose oder Carboxymethylstärke und Natriumsalzen schwacher Säuren in Form einer viskosen und wässrigen Lösung als Ersatz für die amniotische Flüssigkeit zur Schmierung des Geburtskanals bei Säugetieren inklusive Menschen einzusetzen, wobei gemäss der Offenbarung der amniotischen Flüssigkeit eine schmierende Wirkung zugeschrieben wird. Es ist jedoch bekannt, dass Mittel auf wässriger Basis bezüglich Schmierung eine nur geringe Wirkung entfalten. Die Zusammensetzung ist wegen des Gehaltes an Metaphosphatsalzen zudem zumindest für den Menschen physiologisch bedenklich.

Des weiteren ist seit langem bekannt, dass aufgrund der fehlenden Gleitfähigkeit der Geburtskanaloberfläche zur vaginalen Untersuchung bei der gynäkologischen Routineuntersuchung vorgeburtlich oder unter der Geburt ein Schmiermittel verwendet werden sollte. Die Verwendung eines Schmiermittels dient hierbei zur Erleichterung der manuellen vaginalen Untersuchung und nicht zur Geburtserleichterung (Krantz K. E., Gorton, C. J., Obstet. Gynecol. 1973; 41(2), Seiten 308 bis 309).

Durch die technologischen Fortschritte der letzten Jahrzehnte in der Geburtshilfe, in der Betreuung von schwangeren Frauen, der Geburtsleitung und der Neonatologie konnte die kindliche und mütterliche Morbidität und Mortalität entscheidend verbessert werden. Diese Fortschritte haben jedoch auch zu einer Erhöhung der Rate an anästhesiologischen Eingriffen, der vaginaloperativen Eingriffe und der Sectio Caesarea geführt, welche wiederum zu einer Erhöhung der kindlichen und/oder mütterlichen Morbidität führen können.

Aufgrund des legitimen Anspruchs einer schwangeren Frau auf eine sanfte, technologisch zurückhaltende Geburt wurden in den letzten Jahren neue Geburtskonzepte entwickelt und eingeführt. Diese beinhalten vornehmlich ein passives Geburtsmanagement, den restriktiven Einsatz invasiver Methoden und die Wahlfreiheit des Geburtsmodus wie Wassergeburt und alternative Geburtsstellungen. Obwohl diese Konzepte das Geburtserlebnis bei vergleichbarer Morbiditäts- und Mortalitätsrate verbessert haben, besteht nach wie vor ein grosses und bisher unerfülltes Bedürfnis seitens der Schwangeren, die Geburt und insbesondere den vaginalen Geburtsvorgang zu erleichtern und somit das Geburtserieben zu verbessern und Geburtstrauma wie Schädigung des Beckenbodens zu vermindern.

Die geburtsmechanische Bedeutung der Reibungskraft zwischen dem Fötus und dem Geburtskanal ist bis heute bei Humangeburten übersehen worden. Die wirksame Verminderung dieser Reibung ist bei der Humangeburtshilfe unbekannt. Es sind auch noch keine pharmazeutischen Formulierungen zur Verminderung der Reibung für die Geburtshilfe beim Menschen entwickelt oder gar eingeführt worden.

Es wurde nun überraschend erkannt, dass die Humangeburt erheblich erleichtert und sogar zeitlich verkürzt werden kann, insbesondere bei Erstgebärenden, wenn man vor und/oder während der Geburt die Wände von Gebärmuttermund und Vagina (Geburtskanal) mit einem physiologisch unbedenklichen organischen Gleitmittel bedeckt. Mit dem Gleitmittel kann sowohl in der Eröffnungsperiode als auch in der Austreibungsperiode die Reibung zwischen Geburtskanal und Geburtsobjekt stark vermindert werden. Zudem kann die Gefahr von Verletzungen (wie zum Beispiel Auswalzung des Weichteilrohres, Beckenbodenschädigung, Vaginalrisse, Perinealverletzungen, rektale Verletzungen, Uterusrupturen, Blutverlust) vermindert oder ausgeschlossen werden, und Langzeitschäden wie zum Beispiel Urininkontinenz, Stuhlinkontinenz, sexuelle Dysfunktion und psychische Störungen eingeschränkt oder verhindert werden. Ferner kann durch die geringere Reibung die Geburtsarbeit reduziert werden, was zu einer Verhinderung oder Verminderung vaginaloperativer Eingriffe oder Kaiserschnitte führen kann.

Gegenstand der Erfindung ist die Verwendung einer physiologisch unbedenklichen organischen Substanz zur Herstellung einer Zusammensetzung ohne Gehalt an Alkalimetallsalzen von Metaphosphaten zur Anwendung als Gleitmittel bei Vaginalgeburten von Frauen.

Die Wirkung als Gleitmittel wird im Rahmen der Erfindung durch das Einbringen der erfindungsgemäss zu verwendenden Zusammensetzung in den Geburtskanal erzielt. Hierdurch wird eine Gleitschicht zwischen vorzugsweise der gesamten Oberfläche des Geburtskanals und dem Fötus gebildet. Mit der erreichten Verminderung der Reibung (unabhängig ob es sich um Haftreibung, Gleitreibung und/oder Rollreibung handelt) zwischen Fötus und Oberfläche des Geburtskanals wird die Geburt erheblich erleichtert und beschleunigt.

Physiologisch unbedenklich bedeutet im Rahmen der Erfindung, dass die verwendeten Gleitmittel für die Gebärende wie für das Kind verträglich sind.

Organische Substanz bedeutet im Rahmen der Erfindung eine organische Substanz, mit der Gleitmittel hergestellt werden können.

Das Einbringen der erfindungsgemäss zu verwendenden Zusammensetzung erfolgt vorteilhaft vor Geburtsbeginn und/oder in der Eröffnungsperiode und/oder in der Austreibungsperiode. Hierdurch kann auch eine Reibung der ungeöffneten Fruchtblase mit dem Geburtskanal reduziert werden.

Organische Substanzen zur Herstellung von Gleitmitteln sind vielfach bekannt und werden in der Medizin für die verschiedensten Zwecke schon angewendet. Die organischen Substanzen können selbst die gleitende Wirkung in der Zusammensetzungen bewirken (zum Beispiel Fette und Öle, die selbst eine gleitende Wirkung aufweisen), oder sie können durch die Formulierung der Zusammensetzung eine gleitende Wirkung verleihen (zum Beispiel Gele), wobei solche Formulierungen zusätzlich organische Substanzen enthalten können, die selbst eine gleitende Wirkung aufweisen. Die Zusammensetzungen können auch im wesentlichen sphärische Mikropartikel enthalten, um die Verminderung der Reibung durch einen Rolleffekt der Partikel zu nutzen. Die Zusammensetzungen können auch nach dem Einbringen einen Gleitfilm auf der Oberfläche des Geburtskanals ausbilden, der eine höhere Haftung zur Oberfläche des Geburtskanals im Vergleich zur Haut des Fötus besitzt.

Bei den organischen Substanzen mit Gleitwirkung kann es sich zum Beispiel um natürliche oder synthetische Öle, Fette und Wachse handeln, wie zum Beispiel flüssige bis viskose Kohlenwasserstoffe oder Paraffine, Pflanzenöle und -fette, hydrierte oder unhydrierte Fettsäureester. Natürliche Öle, Fette und Wachse können sowohl pflanzlichen als auch tierischen Ursprungs sein. Es kann sich auch um längerkettige Alkohole und deren Ester (Fettalkohole und deren Ester), Polyole (Glycerin oder Saccharide) sowie Tenside handeln.

Bei den organischen Substanzen mit Gleitwirkung kann es sich auch um lösliche, emulgierbare, dispergierbare, gegebenenfalls niedermolekulare, gegebenenfalls biologisch abbaubare und/oder gegebenenfalls bioadhäsive organische Oligomere oder Polymere handeln, die als Gleitfilme bildende oder als Gleitmittel wirkende Zusammensetzungen formuliert werden können, zum Beispiel mit organischen Lösungsmitteln, Wasser oder Mischungen von Lösungsmitteln und Wasser als Träger. Geeignete Lösungsmittel sind zum Beispiel Ether, Alkohole und Polyole, besonders Diole und Triole, wie Ethanol, Ethylenglykol, Propylenglykol, Diethylenglykol, Ethylenglykolmonomethyl- oder -dimethylether, Ethylenglykolmonoethyl- oder -diethylether, Diethylenglykolmonomethyl- oder -dimethylether, Diethylenglykolmonoethyl- oder -diethylether, und Glycerin. Geeignet sind auch Ester wie zum Beispiel Ethylencarbonat und Propylencarbonat.

Organische Substanzen, die durch Formulierung der Zusammensetzung eine gleitende Wirkung verleihen, sind hauptsächlich mit organischen Lösungsmitteln und/oder Wasser quellbare Polymere, die Gele bilden. Bevorzugt sind organische Substanzen, die Hydrogele bilden. Für die Bildung von Gelen können auch mehr als ein unterschiedliches Polymer eingesetzt werden. Polymere für die Formulierung von Gelen können auch selbst eine Gleitwirkung besitzen. Ferner können Gelen organische Substanzen mit Gleitwirkung zugesetzt werden.

Die Zusammensetzungen mit Gleitwirkung können eine ölige, wachsartige, pastöse, schleimige oder fettige Konsistenz aufweisen oder es kann sich um Gele handeln, insbesondere um Hydrogele. Es kommen aber auch Emulsionen oder Dispersionen in Betracht. Weitere Formen möglicher Formulierungen sind zum Beispiel Öle, Pasten, Cremes, Suppositorien oder Schäume. Die Art der Galenik hat nur einen unerheblichen Einfluss auf die Wirkung, kann aber das positive Empfinden der Gebärenden bei der Anwendung beinflussen.

Die organischen Substanzen können alleine oder in Mischung von mindestens zwei Substanzen vorliegen. Ferner ist es zweckmässig, die Substanzen so zu formulieren, dass man einfach anzuwendende Formen wie zum Beispiel viskose Öle, Pasten, Gele, Cremes, Suppositorien oder Schäume erhält, die gleichmässig und vollständig auf der Oberfläche des gesamten Geburtskanals verteilt werden können. Mit einer Formulierung kann zudem die Haftung auf der Oberfläche des Geburtskanals verbessert und gegebenenfalls zusätzlich auf der Fötushaut vermindert werden, so dass eine optimale Dauer und Gleitwirkung der auf der Oberfläche des Geburtskanals aufgebrachten Gleitschicht erzielt wird.

Die Herstellung von Ölen, Pasten, Gelen, Cremes, Suppositorien oder Schäumen sowie Lösungen, Emulsionen und Dispersionen für medizinische Zwecke und zu verwendende Bestandteile für solche Formulierungen sind bekannt und brauchen hier nicht näher erläutert werden. Neben den Gleitmitteln werden als Hilfsmittel zum Beispiel Tenside und Dispergiermittel, Verdickungsmittel, pH-Puffer zur Einstellung eines physiologischen pH Wertes, Lösungsmittel, Träger, Füllstoffe und Konservierungsmittel eingesetzt. Die organische Substanz kann in der Formulierung je nach Wirksamkeit in einer Menge von 1 bis 99 Gew.-%, bevorzugt 2 bis 95 Gew.-%, bevorzugter 5 bis 90 Gew.-%, und besonders bevorzugt 5 bis 80 Gew.-% vorliegen, bezogen auf die Formulierung.

Als organische Substanzen mit Gleitwirkung kommen zum Beispiel in Frage:
a) pflanzliche und tierische Speiseöle und -fette, pflanzliche und tierische Wachse, Monobis Polyester langkettiger, gesättigter oder ungesättigter Carbonsäuren und Alkoholen oder Polyolen wie zum Beispiel Glycerin, Pentaerythrit, Trimethylolpropan oder Sacchariden, Fettalkohole und Carbonsäureester davon, sowie Mineralöle, Mineralfette und Mineralwachse (Paraffine);
b) natürliche, natürliche und modifizierte, oder synthetische Polymere, wie zum Beispiel Cellulose, Kollagen, Hydroxyalkylcellulosen (Hydroxyethylcellulose, Hydroxypropylcellulose), Carboxymethylcellulose und deren Alkalimetallsalze, Celluloseacetat, Hydroxyalkylstärke (Hydroxyethylstärke, Hydroxypropylstärke), Gelatine, Dextran und hydroxyalkylierte Dextrane, Alginate, Polyester von Hydroxycarbonsäuren (Polylactat, Polyglykolat, Polysorbat und Copolymere der entsprechenden Monomeren), aliphatische Polycarbonate (Polybutylencarbonat), Polyalkylenoxide (Polyethylenoxid, Polypropylenoxid, Polyethylen-/Polypropylenoxid-Copolymere), Polyvinylalkohol und Polyvinylpyrrolidon, Hyaluronsäure oder dessen Alkalimetallsalze, Poly(meth)acrylsäure, Poly(meth)acrylsäurealkylester, Poly(meth)acrylsäurehydroxyalkylester, Polyacrylamide, Polyvinylester (Polyvinylacetat), Olefin/Vinylacetat Copolymere, Polyurethane, Polyamide, Polysiloxane (Silicone), Polyester von Dicarbonsäuren und Diolen, und Polyether.

Spezifische Beispiele für organische Substanzen als Gleitmittel sind: Hydroxyethylcellulose, Glycerin, Polycarbophil, Carbopole (beispielsweise Carbopol 907, Carbomer 934P), Hyaluronsäure und deren Salze, succinylierte Gelatine, Paraffinium liquidum, Vaselinum album, Polyethylen- und Polypropylenglykole und Polyethylen-/Polypropylen-Copolymere (Pluronic 127), Dimethicon, Dimethiconol, CyGomethion, Pflanzenöle und -fette, Tieröle und -fette, Mineralöle und -fette, oberflächenaktive Substanzen (Tenside wie Nonoxynol-9, Phospholipide, pulmonale Surfactants wie Lucinactant, Beractant, Phospholipida e pulmone suis).

Zu erwähnen sind auch Polymere, die nach der Applikation einen Gleitfilm bilden, zum Beispiel Hydroxyethylcellulose, Hydroxypropylcellulose, Hydroxypropylmethylcellulose, Polyvinylalkohol, Polyethylenglykole, Polypropylenglykole und Ethylenoxid/Propylenoxid-Copolymere.

Organische Substanzen, die durch Formulierung der Zusammensetzung eine gleitende Wirkung verleihen, sind zum Beispiel: Poly(meth)acrylsäure, Poly(meth)acrylsäurehydroxyethylester, Polyacrylsäureglycerylester, Pol(meth)acrylamide, Polyvinylpyrrolidon, Carboxymethylcellulose, Substanzen menschlichen oder tierischen Ursprungs wie beispielsweise Inhaltsstoffe der Vemix caseosa, des Amnions, Bestandteile der Plazenta, Substanzen ähnlich der pulmonalen Surfactants (beispielsweise Colfoscerilpalmitat, Lucinactant, Beractant, Phospholipida e pulmone suis), und Inhaltsstoffe der Synovialflüssigkeit und des Auges.

Weitere spezifische Beispiele sind Polyaaylsäure, Carboxymethyl Cellulose (MG 50'000 bis 700'000, Substitutiongrad 0,5 bis 1,5), Polyvinylpyrrolidon (MG 5'000 bis 150'000), Carbopole in Konzentrationen von 0,25 bis 5 Gew.-% (Polyacrylsäure) bei Kombination mit Glycerin und NaCl, Cellulosen insbesondere in Konzentrationen von 1 bis 3 Gew.-% sowie in Kombination mit Feuchthaltemitteln und isotonisierenden Substanzen, Johannisbrotkernmehle insbesondere in Konzentrationen von 0,5 bis 3 Gew.-% sowie in Kombination mit Feuchthaltemitteln und isotonisierenden Substanzen.

Es gibt weltweit im Handel kein (registriertes) Produkt für die beschriebene Indikation, nämlich der Reduktion der Reibung während der Vaginalgeburt beim Menschen.

Es sind jedoch Produkte im Handel erhältlich, die für diese Indikation, nämlich der Schmierung des Geburtskanals, teilweise geeignet sein können, obwohl sie für die erfindungsgemässe Anwendung nicht wirkungsoptimiert sind. Diese Produkte werden zum Einreiben von Fingern für die tastende Untersuchung von Körperöffnungen (zum Beispiel der Vagina), Verbesserung des vaginalen Trockenheitsgefühls, Reduktion der Reibungskräfte bei diagnostischen Interventionen (Bronchoskopie), und anderen Indikationen angeboten und verwendet. Beispiele für solche Produkte sind:
a) Aus der Humanmedizin: Instillagel® , Endosgel® , K-Y® ; K-Y® -Produktfamilie (wie KY Ultragel® und andere), Replens® , Mucogyn® , Echovist® , Levovist® , LAM IPM™ Personal Lubricant® , CoLiqufilm® , Very Private™, Trojan™, Vagisil™, Wet® , Astroglide® , ID Millenium® und ID® Familie, Pre-Seed® , Eros™, Divine No. 9® , Probe® , Surgilube® , Lubrajel® , Surfaxin® , Curosurf® , Exosurf® , Survanta® , Gyne-Moistrin™, Ceylor Gel® ;
b) aus der Veterinärmedizin: Vetagel® , Celagel® , Bovivet Gel® , Degragel® .

In einer bevorzugten Ausführungsform kann die erfindungsgemäss zu verwendende Zusammensetzung zusätzlich einen pharmazeutischen Wirkstoff oder eine Kombination von wenigstens zwei pharmazeutischen Wirkstoffen enthalten, die als Medikamente für bestimmte bei der Geburt auftretenden Indikationen dienen, zum Beispiel Geburtshemmung oder Geburtsförderung, Schmerzlinderung, und Verhinderung von Infektionen. Die Menge an pharmazeutischen Wirkstoffen kann zum Beispiel 0,0001 bis 10 Gew.-%, bevorzugt 0,01 bis 10 Gew.-%, und besonders bevorzugt 0,01 bis 5 Gew.-% betragen, bezogen auf die Zusammensetzung.

Einige Beispiele für geburtseinleitende Substanzen sind Oxytocin, Dinoproston, Sulproston, Misoprostol, und Hyaluronidase.

Einige Beispiele für geburtshemmende Substanzen sind Chondroitinsulfat, Hexoprenalin, Fenoterol, Magnesiumsulfat, Atosiban, Kalziumantagonisten, und Nitroglycerin.

Einige Beispiele für schmerzstillende Substanzen sind Ethylchlorid, Bupivacain, Carbostesin, Lidocain, Mepivacain, Rapidocain, Scandicain, Solarcain, Xylesin, und Xylocain.

Einige Beispiele für Desinfektiva sind antibakterielle und antimikrobielle Substanzen wie quartäre Ammoniumverbindungen, Povidon-Jod, und Jod sowie lod-haltige Verbindungen.

Besonders vorteilhaft ist die Beimischung antiviraler Substanzen zum Beispiel gegen die Übertragung von Herpes oder HIV von der Mutter auf das Kind, zum Beispiel Nukleosid Analoga, Nukleosidale Reverse Transkriptase Hemmer, Nicht Nukleosidale Reverse Transkriptase Hemmer, und Protease Inhibitoren.

Als zweckmässig hat sich auch die Beimischung von pulmonalen oberflächenaktiven Substanzen pulmonale Surfactants) eniviesen, mit denen die Atmungstätigkeit des Neugeborenen nach der Geburt erleichtert werden kann, zum Beispiel Colfoscerilpalmitat, Lucinactant, Beractant, Phospholipida e pulmone suis, Perfluorocarbone. Ferner können Substanzen enthalten sein, die die Zervix uteri reifen lässt, zum Beispiel Dinoproston, Sulproston, Misoprostol, und Hyaluronsäure.

Die erfindungsgemäss zu verwendende Zusammensetzung befindet sich vorteilhaft in einer Verpackung, zum Beispiel Dosen oder besser Tuben, oder sie kann als Vaginalowlum angewendet werden. Des weiteren wird vorzugsweise ein vaginaler Applikator eingesetzt. Bei Verwendung von Dosen kann die Zusammensetzung mit Fingern oder Spachteln auf der Oberfläche des Geburtskanals aufgebracht werden. Zweckmässiger sind Tuben, aus denen die Zusammensetzung mittels Druck auf der Oberfläche des Geburtskanals aufgetragen werden können. Die Grösse der Verpackung kann so gewählt werden, dass die Menge der Zusammensetzung für eine einmalige Applikation genügt. Die Tuben können mit einer Verlängerung versehen sein, die im wesentlichen der Länge des Geburtskanals entspricht, an deren Ende die Austrittsöffnung für die Zusammensetzung angebracht ist. Die Austrittsöffnung ist zweckmässig so ausgestaltet, zum Beispiel als runde Spaltöffnung, dass die Zusammensetzung vollständig und im wesentlichen gleichmässig auf der Oberfläche des Geburtskanals verteilt werden kann.

Die Anwendung der erfindungsgemäss zu verwendenden Zusammensetzung gestaltet sich einfach und ist wirkungsvoll, wenn die Zusammensetzung vor Einsetzen der Geburt, in der Eröffnungsphase, oder in der Austreibungsphase appliziert wird. Die Applikation kann ein- oder mehrmals erfolgen. Eine Applikation kurz vor oder in der Eröffnungsphase kann den Vorteil bieten, dass das Gewebe im Geburtskanal erweicht, wodurch die Geburt zusätzlich erleichtert wird.

Ein anderer Gegenstand der Erfindung ist ein Verfahren zur Erleichterung der vaginalen Geburt bei Frauen, bei dem eine Zusammensetzung, enthaltend ein physiologisch unbedenkliches organisches Gleitmittel und keine Alkalimetallsalze von Metaphosphaten, in einer wirksamen Menge in den Geburtskanal von Frauen eingebracht wird.

Wirksame Menge bedeutet im Rahmen der Erfindung eine Volumenmenge von 1 bis 500 ml, bevorzugt 5 bis 200 ml und besonders bevorzugt 10 bis 100 ml. Die Volumenmenge kann von der Art der Formulierung abhängen.

Die erfindungsgemäss zu verwendende Zusammensetzung bietet eine Reihe von Vorteilen und Verbesserungen, an die der Fachmann bislang gar nicht gedacht hat.

Primäre Verbesserungen sind zum Beispiel:
- eine Reduktion der Geburtsarbeit und/oder der Geburtsdauer,
- eine Reduktion des Geburtsschmerzes,
- eine Reduktion der Geburtstraumen bei Mutter und Kind,
- eine Reduktion das peripartalen und neonatalen Infektionsrisiko durch Kombination des Gleitmittels mit antimikrobiellen Substanzen.

Weitere Verbesserungen für die Mutter sind zum Beispiel:
- Vermeidung und/oder Reduktion von Geburtskanalverletzungen der Mutter (Zervixriss, Vaginalriss, Dammriss, Labienriss, Vulväre Verletzungen),
- Prävention von Beckenbodenschädigungen,
- Prävention der Urininkontinenz, Stuhlinkontinenz und psychischen Störungen,
- Reduktion der Episiotomie Rate
- Reduktion und/oder Vermeidung von vaginaloperativen Eingriffen oder der Sectio Caesarea Rate,
- Reduktion der Episiotomie Rate
- Reduktion und/oder Vermeidung von anästhesiologischen Interventionen (Lokal-, Redionalanästhesie, Sedation, Inhalationsnarkose),

Weitere Verbesserungen für das Kind sind zum Beispiel:
- Vermeidung und/oder Reduktion fetaler subpartaler Hypoxien,
- Vermeidung und/oder Reduktion der fetalen Geburtsasphyxie und deren Folgen,
- Vermeidung und/oder Reduktion von kindlichen Geburtsverletzungen wie Claviculafraktur, Plexusläsionen oder Hirnblutungen
- eine Reduktion neonataler Atemnotsyndrome durch Kombination des Gleitmittels mit Substanzen ähnliche der pulmonalen Surfactant
- eine Reduktion der Infektionsraten Mutter zu Kind bei bakteriellen oder viralen Infektionen.

Allgemein kann festgestellt werden, dass das Geburtserlebnis verbessert wird, sowie Gesundheitskosten durch Prävention von Geburtskomplikationen und Langzeitfolgeschäden reduziert werden können.

Das nachfolgende Beispiel erläutert die Erfindung näher.

### Beispiel:

Aus der Literatur ist bekannt, dass die statistische Dauer der Eröffnungsperiode bei Erstgebärenden 600 Minuten, der Austreibungsperiode 120 Minuten und die Gesamtzeit somit 720 Minuten beträgt (Normalkollektiv).

Als Gleitmittel wird im Einverständnis mit den Gebärenden das Gel KYR Gleitgel® der Firma Johnson & Johnson verwendet. Dieses Gleitmittel wird für tastende Vaginaluntersuchungen eingesetzt, um Finger der untersuchenden Person gleitfähiger zu machen. Das Gleitmittel ist für den verwendeten Zweck, nämlich die Erleichterung der Geburt durch Einbringen in den der Geburtskanal, nicht bekannt und nicht optimiert.

Es werden acht Erstgebärende für ein gleiches Geburtsteam selektiert. Das Gel wird in solchen Mengen während der Eröffnungsperiode und der Austreibungsperiode angewendet, dass möglichst die gesamte Oberfläche des Geburtskanals stets ausreichend mit dem Gel bedeckt ist (insgesamt etwa 80 g Gel, diese Menge ist bei weitem höher als die Menge, die zur vaginalen Untersuchung unter der Geburt verwendet wird). Bei sechs Gebärenden platzt die Fruchtblase innerhalb 30 Minuten vor Ende der Eröffnungsperiode.

Bei einem mittleren Geburtsgewicht von 3189 ± 326 g, einem mittleren Kopfumfang von 34 ± 1,1 cm, und einer mittleren Körperlänge von 47,6 ± 1,8 cm beträgt die mittlere Dauer der Geburt insgesamt 336 ± 184 Minuten, wobei eine signifikante zeitliche Verkürzung der Geburtsdauer beobachtet wird. Die neonatalen Outcome Parameter (APGAR, pH der Nabelschnur) sind vergleichbar mit dem Normalkollektiv.

Die subjektive Beurteilung des Geburtsvorgangs des Geburtsteams und der Gebärenden ist in allen Fällen positiv, obwohl ein nicht optimiertes Produkt verwendet wurde.

## Patentansprüche

1. Verwendung einer physiologisch unbedenklichen organischen Substanz zur Herstellung einer pharmazeutischen Zusammensetzung ohne Gehalt an Alkalimetallsalzen von Metaphosphaten zur Anwendung als Gleitmittel bei Vaginalgeburten von Frauen, wobei
die organische Substanz selbst die gleitende Wirkung in der pharmazeutischen Zusammensetzung bewirkt oder die organische Substanz durch die Formulierung der pharmazeutischen Zusammensetzung eine gleitende Wirkung verleiht, und wobei es sich bei der organischen Substanz mit Gleitwirkung um lösliche, emulgierbare, dispergierbare, gegebenenfalls niedermolekulare, gegebenenfalls biologisch abbaubare und/oder gegebenenfalls bioadhäsive organische Oligomere oder Polymere handelt, die als gegebenenfalls Gleitfilme bildende oder als Gleitmittel wirkende Zusammensetzungen formuliert worden sind, oder wobei es sich bei der organischen Substanz, die durch Formulierung der pharmazeutischen Zusammensetzung eine gleitende Wirkung verleiht, um mit organischen Lösungsmitteln und/oder Wasser quellbare Polymere handelt, die Gele bilden, und
die pharmazeutische Zusammensetzung in Form von Pasten, Gelen (Hydrogelen), Cremes oder Schäumen vorliegt.

2. Verwendung nach Anspruch 1,
**dadurch gekennzeichnet,**
**dass** die organische Substanz in einer Menge von 1 bis 99 Gew. -% in der pharmazeutischen Zusammensetzung enthalten ist.

3. Verwendung nach Anspruch 1 oder 2,
**dadurch gekennzeichnet,**
**dass** die pharmazeutische Zusammensetzung zusätzlich einen pharmazeutischen Wirkstoff oder eine Kombination von wenigstens zwei pharmazeutischen Wirkstoffen enthält.

4. Verwendung nach Anspruch 3,
**dadurch gekennzeichnet,**
**dass** die Menge an pharmazeutischen Wirkstoffen 0,0001 bis 10 Gew. - % beträgt, bezogen auf das Gleitmittel beziehungsweise die Formulierung mit einem Gleitmittel.

5. Verwendung nach Anspruch 3 oder 4,
**dadurch gekennzeichnet,**
**dass** die pharmazeutischen Wirkstoffe der Geburtshemmung oder Geburtsförderung, der Schmerzlinderung, der Verhinderung von Infektionen oder der Prävention des Atemnotsyndroms beim Neugeborenen dienen.

6. Verwendung gemäß einem der Ansprüche 1 bis 5,
**dadurch gekennzeichnet,**
**dass** die physiologisch unbedenkliche Substanz Polyacrylsäure, insbesondere in Konzentrationen von 0,25 bis 5 Gew.-%, ist.

7. Verwendung nach Anspruch 6,
**dadurch gekennzeichnet,**
**dass** die pharmazeutische Zusammensetzung zusätzlich Feuchthaltemittel und isotonisierenden Substanzen enthält.

8. Verwendung nach einem der Ansprüche 1 bis 7,
**dadurch gekennzeichnet,**
**dass** die pharmazeutische Zusammensetzung in einer Menge von 1 bis 500 ml zur Anwendung gelangt.

9. Verwendung nach einem der Ansprüche 1 bis 8,
**dadurch gekennzeichnet,**
**dass** die pharmazeutische Zusammensetzung vor Einsetzen der Geburt, in der Eröffnungsphase oder in der Austreibungsphase zur Anwendung gelangt.

10. Verwendung nach einem der Ansprüche 1 bis 9 zur Reduktion der Geburtsarbeit der Mutter, der Geburtsdauer bei der Mutter, des Geburtsschmerzes bei der Mutter, der Geburtstraumen bei Mutter und Kind, der Episiotomie-Rate bei der Mutter, des peripartalen und neonatalen Infektionsrisikos bei der Mutter, von neonatalen Atemnotsyndromen beim Kind, der Infektionsraten Mutter zu Kind, zur Vermeidung und/oder Reduktion von Geburtskanalverletzungen bei der Mutter, von vaginaloperativen Eingriffen oder der Sectio Caesarea-Rate bei der Mutter, von anästhesiologischen Interventionen bei der Mutter, der fetalen subpartalen Hypoxie beim Kind, der fetalen Geburtsasphyxie und deren Folgen beim Kind, von Geburtsverletzungen beim Kind, und zur Prävention von Beckenbodenschädigungen bei der Mutter, von Urininkontinenz, Stuhlinkontinenz und psychischen Störungen bei der Mutter.

11. Nicht-therapeutisches Verfahren zur Erleichterung der vaginalen Geburt bei Frauen, bei dem eine Zusammensetzung in Form von Pasten, Gelen (Hydrogelen), Cremes oder Schäumen, enthaltend ein physiologisch unbedenkliches organisches Gleitmittel und keine Alkalimetallsalze von Metaphosphaten, in wirksamer Menge in den Geburtskanal von Frauen eingebracht wird.

12. Nicht-therapeutisches Verfahren nach Anspruch 11,
**dadurch gekennzeichnet,**
**dass** das organische Gleitmittel selbst die gleitende Wirkung in der Zusammensetzung bewirkt oder das organische Gleitmittel durch die Formulierung der Zusammensetzung eine gleitende Wirkung verleiht, und wobei es sich bei dem organischen Gleitmittel mit Gleitwirkung um lösliche, emulgierbare, dispergierbare, gegebenenfalls niedermolekulare, gegebenenfalls biologisch abbaubare und/oder gegebenenfalls bioadhäsive organische Oligomere oder Polymere handelt, die als gegebenenfalls Gleitfilme bildende oder als Gleitmittel wirkende Zusammensetzungen formuliert worden sind, oder wobei es sich bei dem organischen Gleitmittel, das durch Formulierung der Zusammensetzung eine gleitende Wirkung verleiht, um mit organischen Lösungsmitteln und/oder Wasser quellbare Polymere handelt, die Gele bilden.

13. Nicht-therapeutisches Verfahren nach Anspruch 11 oder 12,
**dadurch gekennzeichnet,**
**dass** die Zusammensetzung in einer Menge von 1 bis 500 ml in den Geburtskanal von Frauen eingebracht wird.

14. Nicht-therapeutisches Verfahren nach einem der Ansprüche 11 bis 13,
**dadurch gekennzeichnet,**
**dass** die Zusammensetzung vor Einsetzen der Geburt, in der Eröffnungsphase oder in der Austreibungsphase in den Geburtskanal von Frauen eingebracht wird.
